# EUROPEAN PATENT APPLICATION

(11) **EP 3 034 077 A1**
(43) Date of publication of application: **22.06.2016**
(21) Application number: 15199663.4
(22) Date of filing: 11.12.2015
(51) Int. Cl.: A61K 31/43, A61K 31/4439, A61K 31/7048, A61P 1/04, A61P 31/04

(54) **KIT FOR CO-ADMINISTRATION OF ESOMEPRAZOLE, AMOXICILLIN AND CLARITHROMYCIN**

(30) Priority: 15.12.2014 BR 202014031413 U
(71) Applicant: EMS S.A., CEP-13186-901 Hortolandia, SP (BR)
(72) Inventor: DA CUNHA ECCLISSATO, Christina, 13186-901 São Paulo (BR); DE PAULA MACHADO JÚNIOR, Celso, 13186-901 São Paulo (BR); KHATER COVESI, Leticia, 13186-901 São Paulo (BR); TIROLI CEPEDA, Ana Oíivia, 13186-901 São Paulo (BR)
(74) Representative: Cabinet Plasseraud

(57) **Abstract**

Presented in this invention, belonging to the pharmaceutical field, a facilitator kit for the co-administration of drugs for the treatment of *Helicobacter pylori* infections.

## Description

### FIELD OF APPLICATION

The present invention relates to the pharmaceutical field, specifically to a device to facilitate the co-administration of drugs with application in the treatment of *Helicobacter pylori* infections.

### BACKGROUND

*Helicobacter pylori* infection has related occurrences around the world, with higher incidence in developing countries. According to figures updated in 2010 by the World Gastroenterology Organization, more than 50% of the population is afflicted with infection by the bacterium, while in Brazil the prevalence rate in adults is 82%. Since its discovery, several studies have been developed aiming at its eradication.

The first reports related to *H. pylori* date back to 1875, when German scientists reported the presence of spiral bacteria in the gastric mucosa of humans. However, since the researchers were unable to grow such bacteria in vitro, the results were ignored. Over the next one hundred years, little was discovered, until, in 1982, Australian doctors Barry J. Marshall and J. Robin Warren were able to isolate the bacteria in order to carry out studies (Blaser, M.J. "An Endangered Species in the Stomach" Scientific American, 2005, 292: 38-45).

In their trials, Marshall and Warren discovered a correlation between the presence of the bacteria in the gastric tract with the incidence of peptic ulcers. Subsequently, a relationship was found between *H. pylori* and the appearance of gastric carcinoma (Forman, D. et al "Association between infection with Helicobacter pylori and risk of gastric cancer: Evidence from a Prospective Investigation" BMJ 1991; 302: 1302-5).

In 2005, Marshall and Warren were jointly awarded the Nobel Prize for Medicine for their contribution to the discovery of *Helicobacter pylori* and its role in the occurrence of gastritis and peptic ulcer ("The Nobel Prize in Physiology or Medicine in 2005." Nobelprize.org. Nobel Media AB 2014. Web.28 Nov 2014. <http://www.nobelprize.org/nobel_prizes/medicine/laureates/2005/ index.html>).

Currently, in cases of gastric distress, standard testing is the identification of H. pylori in the gastric mucus during endoscopic examination, using, for example, the rapid urease test (RUT) and fluorescence in situ hybridization (FISH). If there is a clinically suspected infection, it is also possible to carry out non-invasive tests such as the stool antigen and serology test (SAT), with the choice of method being determined by such factors as availability of reagents and test cost. With the confirmation of the presence of the bacteria, various treatments can be followed, always aiming at the eradication of *H. pylori* to improve the condition of the patient.

According to the Consensus of Maastricht IV of 2012 (Malfertheiner, P. et al. "Management of Helicobacter pylori infection - the Maastricht IV / Florence Consensus Report" Gut 2012; 61: 646-664), the minimum acceptable rate of *eradication of H. pylori* in first line treatment, i.e. the treatment used after the identification of infection, is 80%.

As commonly occurs in infections treated with antibiotics, the emergence of resistance by *H. pylori* is a critical issue that requires special care and a variety of solutions to overcome it, as described below.

Antibiotics commonly used in the treatment of infections by *H. pylori* include clarithromycin, for which resistance has been reported, for example, by Xia (Xia, HC et al. "Clarithromycin resistance in Helicobacter pylori: prevalence in untreated dyspeptic patients and stability in vitro," J Antimicrob Chemother 1996, 37: 473-481), levofloxacin, for which resistance is rapidly increasing around the world, metronidazole, for which resistance is generally overcome by increasing the applied dose, amoxicillin, which has few cases of resistance, and tetracycline and bismuth which have rare cases of reported resistance (De Francesco, V. et al. "Worldwide H. pylori Antibiotic Resistance: a Systematic Review" J Gastrointestinal Liver Dis, 2010; 19 (4): 409-414).

The types of antibiotic resistance are classified as cross-resistance, which occurs with compounds of the same class of antibiotic, e.g., resistance to levofloxacin and consequent resistance to fluoroquinolones, and no cross-resistance, which occurs with compounds of different classes, for example, resistance to levofloxacin and metronidazole, in which the resistance mechanisms are distinct.

With regard to clarithromycin, its resistance increased from 9% in 1998 (Glupczynski, Y. et al. "European multicenter survey of in vitro antimicrobial resistance in Helicobacter pylori. "Eur J Clin Microbiol Infect Dis 2001; 20: 820-3) to 17.6% in 2008-9 (Megraud, F. et al. "Surveillance of Helicobacter pylori resistance to antibiotics in Europe 2008-2009" Gastroenterology 2011; 140: 1715) in Europe. With regard to Brazil, although there is limited data available, studies have shown a resistance ranging from 8% -16% per product (Eisig, JN "Helicobacter pylori Antibiotic Resistance in Brazil: Clarithromycin is Still a Good Option "Arq. Gastroenterol 2011.; 48 (4): 261-264 and Godoy, AP et al. "Analysis of Antimicrobial Susceptibility and Virulence Factors in Helicobacter pylori Clinical Isolates" BMC Gastroenterol. 2003; 3:20, respectively), demonstrating that its use in multiple therapies is still an alternative for the eradication of *H. pylori.*

The understanding of the technique regarding the resistance of *H. pylori* is that it is the result of mutations which occur at random and may remain for many generations. Usually, a low proportion of these mutant strains are present in a population of *H. pylori* and remain undetectable by methods known in the prior art.

However, when an antibiotic is prescribed for a given infection, there may be a selection of resistant strains while the susceptible strains are killed. It is also known that the indiscriminate use of antibiotics by the population may lead to a selection of resistant strains. Thus, these strains reproduce, becoming the majority of the bacterial population which precludes the use of antibiotics to eradicate said infection (Mégraud F. "The Challenge of Helicobacter pylori Resistance to Antibiotics "Ther Adv Gastroenterol. 2012;5(20: 13-109).

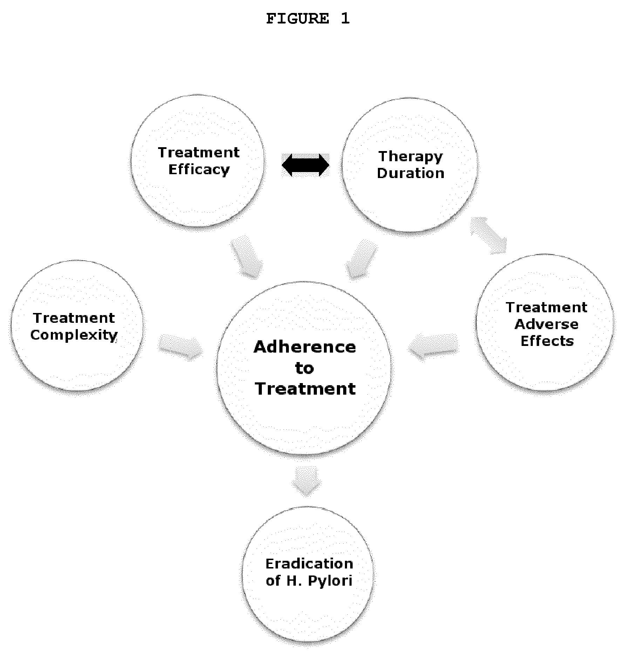

As a result, successful eradication of *H. pylori,* as well as inhibition of formation of resistant strains depends upon full compliance with treatment prescribed by a qualified practitioner, as illustrated in Figure 1. In addition, variables such as dosage and dosage intervals(s) of drug(s), gastric hypersecretion of the treated patient and the installed bacterial load are relevant for effective treatment against *H*. *pylori*.

According to the infectious table below, different levels of treatment may be prescribed, which relate mainly to regional statistics of antibiotic resistance and previous therapeutic failures. They are: the treatment of first-line, second-line and third-line.

In relation to the first-line treatment, approaches are conventional triple therapy, sequential therapy, concomitant therapy and quadruple therapy with bismuth.

The first approach is the most used, having many studies proving its effectiveness and application limitations. It consists of the co-administration of a proton pump inhibitor (PPI) with two antibiotics for a period ranging from 7 to 14 days. Combinations of clarithromycin, metronidazole and a PPI are encompassed by this approach.

Sequential therapy involves two distinct stages of administration, each lasting five days. In the first five days, administration of a PPI combined with amoxicillin is performed, followed by administration of a PPI combined with clarithromycin and metronidazole/tinidazole over the next five days. However, the number of antibiotics used and their change during the administration hamper adherence to treatment, which, as mentioned earlier, is critical to success in the eradication of *Helicobacter pylori.* Additionally, many clinicians fear the emergence of antibiotic resistance to this treatment.

Sequential therapy is very similar to concomitant therapy, with the administration of the three antibiotics taken together throughout the treatment which usually lasts from 10 to 14 days.

Finally, the quadruple therapy with bismuth involves a PPI associated with three antibiotics, with one of them being a bismuth compound. This therapy lasts 10 to 14 days and is considered to be highly efficient with eradication in the range of 92% of cases.

The second-line treatment is performed when a therapeutic failure, i.e., when the first-line treatment is unable to eradicate the bacterial population. The third-line treatment is performed after two therapeutic failures, i.e., when the first and second line therapies are unable to eradicate the infection.

The availability of different first-line treatment alternatives is still, therefore, highly desirable, especially in view of the great variability of resistant strains in different geographic regions. Additionally, the eradication of *H. pylori* with first-line treatments ensures greater stability and comfort for patients under management since the second and third line treatments cause more harmful side effects which undermine adherence by the patient.

### SUMMARY OF THE PRESENT INVENTION

The object of the present invention is to provide a kit for the co-administration of drugs characterized in that said drugs are present in a single pack or cartridge under distinct pharmaceutical forms, in which each product is supplied in a different compartment of said kit, wherein the kit comprises distinguishing marks to facilitate the administration of the drugs which are directed to the treatment of *Helicobacter pylori* infections which comprise a proton pump inhibitor plus two antibiotics, wherein the proton pump inhibitor is esomeprazole and the antibiotics are amoxicillin and clarithromycin.

### DESCRIPTION OF THE FIGURES

Figure 1 is a diagram of factors related to the eradication of *H. pylori* infection, with patient compliance seen as the key parameter for effective treatment. It is further shown that the duration of therapy directly affects therapeutic effectiveness by time of exposure of the bacteria to the drugs, and adverse effects, whose occurrence varies with the time of treatment.

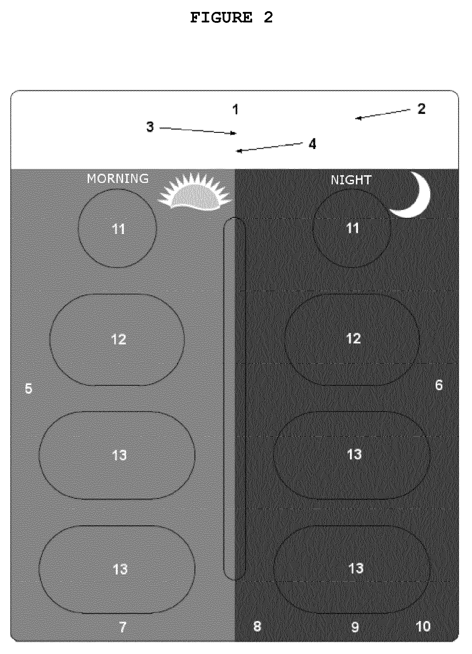

Figure 2 depicts the scheme of the device according to the present invention with indicative numbering as described below. The name of the device for co-administration of drugs to treat *H. pylori* is referred to as 1, the company's brand is arranged on the right side of the product name and is referred to as 2, the list of present drugs (Amoxicillin + Clarithromycin + Magnesium Esomeprazole) is referred to as 3 and their dosages (500 mg + 500 mg + 20 mg or 40 mg) are referred to as 4, indications for administration by the user in the morning and at night are referred to as 5 and 6, respectively, marketing instructions are referred to as 7, the product lot is referred to as 8, the contact for the customer service department (CSD) is referred to as 9, the administration route is referred to as 10, the drug names on each compartment as well as their strengths: magnesium esomeprazole (20 mg or 40 mg), clarithromycin (500 mg) and amoxicillin (500 mg) are referred to respectively as 11, 12 and 13.

### DESCRIPTION OF THE INVENTION

As mentioned above, the present invention consists of a device for the co-administration of drugs for first-line treatment against *Helicobacter pylori* bacteria consisting in a single package, which may be a pack (blister) or cartridge. This kit provides an improvement over the prior art for adherence to treatment.

The drugs comprise a proton pump inhibitor and two antibiotics, with the PPI being esomeprazole and the antibiotics being amoxicillin and clarithromycin. Esomeprazole is presented in an amount of 20-40 mg / dosage form, while the amoxicillin and clarithromycin are each presented in an amount of 500 mg / dosage form. The possible dosage forms of each drug are, independently, capsules, tablets, coated pills, lozenges or pills, in a form that can be immediate or modified release.

The proton pump inhibitor esomeprazole, referred to interchangeably here in the form of magnesium salt, or magnesium, esomeprazole has the advantage compared to its precursor omeprazole of being metabolized quickly by the liver, resulting in higher concentrations of the active ingredient in the circulatory system and consequent greater suppressive action of gastric acidity, leaving the population of *H. pylori* more susceptible to the action of antibiotics (Dent, J. "Review Article: Pharmacology of Esomeprazole and Omeprazole Comparisons with "Aliment Pharmacol Ther 2003; 17 (1) : 5-9).

The kit is composed of several compartments of variable sizes which are coated with a mixed layer of aluminum and polymer material, on one side, and a single layer of aluminum on the other side for the protection of the pharmaceutical forms.

As can be seen by Figure 2, there is a difference in color between the portion of the pack containing the drugs to be administered during the day and the others for administration at night. Along with the user administration indications 5, the arrangement in two colors promotes facility for the kit's administration, so that adherence to treatment, essential for the eradication of *H. pylori* (see Figure 1) takes place without effort by the patient.

It is believed that the efficacy of the co-administration drug kit of the present invention is closely related to the maintenance of a high pH, above 4, for esomeprazole. With this reduction in acidity, the population of *H. pylori* is more susceptible to the action of amoxicillin and clarithromycin, thereby reducing the symptoms of patient gastric pain.

The kit presented herein also has the advantage of drastically reducing treatment costs for the patient, since instead of needing to acquire each drug separately in different quantities required for treatment, the patient may acquire such drugs together in the form of the kit disclosed herein for the treatment against *H. pylori.* This fact is also reflected in patient compliance.

From this description, one realizes the functional improvement provided by this invention in the pharmaceutical field, with the attached complementary figures for a full understanding.

The essential features of this invention are highlighted in the set of claims, in particular in independent claim 1, with incremental changes defined by the dependent claims 2 and 3.

## Claims

1. Kit for the co-administration of drugs **characterized in that** said drugs are present in a single pack or cartridge under different pharmaceutical forms, in which each product is supplied in a different compartment of said kit, wherein the kit comprises distinguishing marks to facilitate the administration of drugs which are directed to the treatment of *Helicobacter pylori* infections and comprise a proton pump inhibitor plus two antibiotics, wherein the proton pump inhibitor is esomeprazole and antibiotics are amoxicillin and clarithromycin.

2. Kit for the co-administration of drugs according to claim 1 **characterized in that** the proton pump inhibitor esomeprazole is present in an amount of 20-40 mg / dosage form, the antibiotic amoxicillin is present in an amount of 500 mg / dosage form and the antibiotic clarithromycin is present in an amount of 500 mg / dosage form.

3. Kit for the co-administration of drugs according to claim 1 **characterized in that** said drugs can be independently presented in the form of capsules, tablets, coated pills, lozenges or pills.
